# EUROPÄISCHE PATENTSCHRIFT

(11) **EP 1 857 127 B1**
(45) Veröffentlichungstag und Bekanntmachung des Hinweises auf die Patenterteilung: **08.11.2017**
(21) Anmeldenummer: 07017424.8
(22) Anmeldetag: 26.08.2003
(51) Int. Cl.: A61L 29/16, A61L 29/08, A61M 25/10

(54) **Ballonkatheter zur Arzneimittelabgabe**
Balloon catheter for drug delivery
Cathéter à ballonnet pour la délivrance de médicament

(30) Priorität: 20.09.2002 DE 10244847
(43) Veröffentlichungstag der Anmeldung: 21.11.2007
(62) Teilanmeldung aus: 03750300.0
(73) Patentinhaber: Bayer Intellectual Property GmbH, 40789 Monheim (DE)
(72) Erfinder: Speck, Ulrich, Prof. Dr., 13465 Berlin (DE); Scheller, Bruno, Dr., 66111 Saarbrücken (DE)
(74) Vertreter: Zimmermann & Partner Patentanwälte mbB

(56) Entgegenhaltungen:
- WO-A-01/83016
- WO-A-92/15282
- WO-A-96/39949
- WO-A1-00/21584
- WO-A1-00/32238
- WO-A1-01/49268
- WO-A1-01/52772
- WO-A1-99/08729
- WO-A1-99/59649
- WO-A1-2004/006976
- US-A- 5 102 402
- US-A- 5 370 614

## Beschreibung

Die Erfindung betrifft einen Ballonkatheter zur Arzneimittelabgabe für die selektive Therapie bestimmter Gewebeabschnitte oder Organteile.

Zahlreiche Erkrankungen betreffen nicht den gesamten Organismus gleichzeitig sondern sind auf bestimmte Gewebearten, häufig auch auf sehr begrenzte einzelne Gewebebezirke oder Organteile beschränkt. Beispiele dafür finden sich unter den Tumor-, Gelenk- und Gefäßerkrankungen.

Die Pharmakotherapie auch dieser Erkrankungen erfolgt im allegemeinen durch orale oder intravenöse Gabe von Arzneistoffen, die sich im ganzen Körper verteilen und in vielen Fällen gerade bei schweren Erkrankungen unerwünschte Wirkungen in gesunden Geweben und Organen verursachen, die die therapeutische Anwendung begrenzen. Eine selektive Therapie der erkrankten Gewebe wurde mittels spezifisch an erkranktes Gewebe bindende Arzneistoffe (z. B. Antikörper) unter Beibeihaltung des Applikationsweges oder durch selektive Verabreichung, z.B. durch direkte Injektion in das erkrankte Gewebe oder durch Zufuhr über Katheter zu den das erkrankte Gewebe versorgenden Blutgefäßen, erreicht. Im Falle der selektiven Verabreichung entstehen durch die meist kurze Wirkdauer der Arzneistoffe und die invasiven Verabreichungswege Probleme, da sich eine beliebig wiederholte Gabe verbietet. Bei selektiver Verabreichung über den das erkrankte Gewebe versorgenden Blutstrom ergibt sich das zusätzliche Problem ungenügender Extraktion der Arzneistoffe bei der raschen Passage des Blutes oder der Wirkstofflösung durch die Blutgefäße. Diesen Problemen wurde bisher durch unterschiedliche pharmazeutische Präparate mit verzögerter Wirkstofffreigabe, arzneimittelfreisetzende Implantate oder für längere Zeit funktionsfähige selektive Zugangswege wie implantierte Katheter etc. begegnet.

Es ist bereits bekannt, die Oberfläche von in den Körper eingebrachten medizinischen Geräten, insbesondere Kathetern, mit Mitteln zur Verbesserung der Gleitfähigkeit oder zur Verhinderung der Blutgerinnung, jedoch ohne therapeutische Wirkung, zu beschichten.

Darüber hinaus werden Katheter mit speziellen Vorrichtungen versehen, um Arzneimittel in die Arterienwand zu injizieren, beispielsweise mittels Nadeln oder hohem Injektionsdruck über eine an der Gefäßwand anliegende Perforation der Katheterwand.

Andere Prinzipien beruhen darauf, die Kontaktzeit zwischen Arterienwand und einer über den Katheter applizierten Wirkstoffzubereitung zu verlängern, indem entweder der Blutstrom für einen entsprechenden Zeitraum unterbunden wird, z. B. Doppelballonkatheter mit zwischen den Ballons befindlicher mit der Arzneistofflösung gefüllter Kammer oder Hohlräumen zwischen der z.B. mit Wülsten versehenen Ballonaußenwand, wobei der Blutstrom durch einen den Ballon passierenden Kanal in begrenztem Maße aufrechterhalten werden kann.

Dokument WO 01/83016 A2 betrifft ein Gerät und Verfahren zur Behandlung von Herzinsuffizienz.

WO 96/39949 betrifft ein System zum Auslösen einer Arzneistofffreisetzung.

WO 01/49268 A1 betrifft pharmazeutische Formulierungen zur Arzneistoffzufuhr sowie eine Katheterbeschichtung.

WO 01/52772 A1 betrifft ein Verfahren und Gerät zum Beschichten einer Endoprothese, wie zum Beispiel eines Gefäßstents.

WO 00/32238 betrifft einen Stent mit Arzneistoffkristallen darauf, welche zum Beispiel gebildet wird durch Lösen eines therapeutischen Mittels in einer Lösung mit einem Polymer und Aufbringen der Lösung auf eine medizinische Vorrichtung und Aussetzen der beschichteten medizinischen Vorrichtung einem Nicht-Lösungsmittel.

WO 99/08729 und WO 00/21584 betreffen das Beladen und Freisetzen von wasserunlöslichen Arzneistoffen, wobei eine medizinische Vorrichtung mit einer Polymerbeschichtung beschichtet wird.

WO 99/59649 betrifft die lokale Verabreichung von Arzneimitteln mit einer medizinischen Vorrichtung, welche ein Substrat und eine Beschichtung auf dem Substrat umfasst, in welche ein Arzneimittel inkorporiert ist.

Gemäß der US 5 102 402 werden Arzneistoffe in Form von Mikrokapseln zur verzögerten Wirkstofffreigabe lose in vorgeformten Vertiefungen von Ballonkathetern untergebracht. Nach Expansion des Ballons sollen die Mikrokapseln in die Gefäßwand gedrückt werden, dort verbleiben und den oder die Wirkstoffe langsam freisetzen. Zahlreiche Autoren schlagen auch vor, Arzneistoffe in Hydrogel eingebettet auf Ballonkatheter aufzubringen, wobei die Funktion des Hydrogels als Haftmittel, zur Verbesserung der Gleitfähigkeit oder Verzögerung der Freisetzung der Arzneistoffe offen bleibt.

Nachteil der genannten Produkte ist in jedem Falle der komplexe Aufbau mit entsprechenden Problemen bei Herstellung, Qualitätskontrolle und Kosten sowie zusätzlichen, Arzt und Patient belastenden Arbeitsschritten bei der Anwendung. Ein Teil der genannten Methoden führt zu einer über die beabsichtigte Gefäßerweiterung hinausgehenden gänzlich unerwünschten Gefäßverletzung. Andererseits hat jede zur Verlängerung der Kontaktzeit vorgesehene Mäßnahme eine zusätzliche Minderversorgung der nachgeschalteten Gewebe mit Blut und Sauerstoff zur Folge.

Der Vollständigkeit halber wird noch auf eine in der WO 01/24866 beschriebenen Vorrichtung zum Verhindern der Restenose verwiesen, die mit einer von natürlichen Zellmembranen abgeleiteten lipiden Ceramide-Substanz beschichtet sind. Diese Substanz wird aufgrund ihrer bei gebräuchlichen Arzneistoffen nicht anzutreffenden Affinität zu den Zellwänden der Arterienwand verwendet. In der Fachliteratur wird jedoch weiterhin die Auffassung vertreten, dass eine Arzneimittelprophylaxe der Restenose eine Freisetzung der erforderlichen Wirkstoffe über Tage erfordert.

Der Erfindung liegt die Aufgabe zugrunde, eine Vorrichtung für eine auf bestimmte Gewebebereiche oder Organteile begrenzte Arzneimittelabgabe bereitzustellen, die ohne schädigenden Einfluss auf gesundes Gewebe eine starke therapeutische Wirkung ausübt, den Patienten nur wenig belastet und mit geringem Aufwand angewendet und hergestellt werden kann.

Erfindungsgemäß wird die Aufgabe mit einer gemäß den Merkmalen des Anspruchs 1 ausgebildeten bzw. hergestellten Vorrichtung gelöst. Aus den Unteransprüchen ergeben sich weitere Merkmale und vorteilhafte Weiterbildungen der Erfindung.

Durch die Erfindung werden in einem einfachen Herstellungsverfahren verbesserte arzneistofftragende Ballonkatheter bereitgestellt, die vielseitig anwendbar sind und eine sofortige Wirkstofffreigabe ermöglichen. Überraschend und entgegen der Lehrmeinung ist keine andauernde Wirkstofffreisetzung aus einer inerten Matrix (Polymer,Hydrogel, Mikrokapseln etc.) oder speziellen chemischen oder physikalischen Zuständen der Wirkstoffe erforderlich oder nützlich. Daher werden auch keine aufwendigen Techniken zur Produktion oder Kontrolle von Depotformulierungen benötigt.

Die Beschichtung von auf Kathetern befindlichen Ballonen mit Arzneistoffen gemäß der vorliegenden Erfindung ist insofern von besonderem Nutzen als nach dem Erweitern von Blutgefäßen oder anderen Hohlräumen im Körper mit den Ballonen häufig der Bedarf an therapeutischen Maßnahmen besteht, um eine Verengung oder einen Verschluss des mit dem Ballon unter Druck geschaffenen Lumens zu verhindern, Tumorwachstum zu begrenzen oder Heilungsprozesse einschließlich der Bildung von Kollateralkreisläufen zu fördern. Dies kann durch Arzneistoffe erreicht werden, die ihre Wirkung in unmittelbarer Nähe der Ballonoberfläche entfalten. Die Arzneistoffe haften auf dem Weg zum Ziel - meist durch intensiv durchblutete Arterien - bis zur Entfaltung des Ballons fest auf diesem, werden dann während der kurzen, oft nur Sekunden andauernden Kontaktzeit des entfalteten Ballons an das Gewebe in wirksamer Dosis abgegeben und von diesem in einer Weise aufgenommen, die das Abspülen durch den nach Deflation des Ballons sofort wieder einsetzenden Blutstrom vermeidet.

Zur Beschichtung sind erfindungsgemäß Katheter bzw. Teile von Kathetern, die zumindest für kurze Zeit mit Druck gegen erkrankte Gewebe gepresst werden, vorgesehen. Bevorzugte Kathetermaterialien sind Polyamide, Polyamid-Gemische und Copolymere, Polyethylenterephthalat, Polyethylen und Copolymere, Polyurethan, Naturkautschuk und dessen Derivate. Die Länge und der Durchmesser der für die Pharmakotherapie vorgesehenen Bereiche der Katheter bzw. Ballone ist für die Anwendung nicht von entscheidender Bedeutung, da die Dosierung in *µ*g Wirkstoff/ mm² Oberfläche berechnet wird. Beispielsweise sind für die Koronardilatationen Ballone im Bereich von 2 - 4 mm Durchmesser und von 1,0-4,0 cm Länge gebräuchlich. Für andere Gefäße können auch Ballone bis zu > 20 mm Durchmesser und Längen bis zu > 10 cm eingesetzt werden. Die zu beschichtenden Oberflächen können glatt (d.h. ohne besondere Struktur zur Aufnahme der Wirkstoffe), aufgeraut oder in beliebiger Weise mit Strukturen versehen sein, wobei spezielle Oberflächenstrukturen nicht Voraussetzung für die Haftung der Wirkstoffe sind, die Haftung aber auch nicht behindern. Die Haftung der Wirkstoffe auf den Ballonoberflächen wird ausschließlich durch die Wahl geeigneter Lösungsmittel und ggf. die Haftung beeinflussende Zusatzstoffe bewirkt. Sie ist selbst auf äußerlich vollständig glatten Ballonoberflächen überraschend fest.

Alle Oberflächen können zusätzlich mit Substanzen beschichtet worden sein oder werden, die die Gleitfähigkeit der Produkte verbessern, die Gerinnung von Blut an der Oberfläche verhindern oder sonstige Eigenschaften der Medizinprodukte verbessern, ohne dass die zur Beschichtung benutzten Materialien an die Umgebung abgegeben werden müssen und ohne dass die Beschichtung die Abgabe der Wirkstoffe zur Behandlung der Zielgewebe und damit die Wirksamkeit wesentlich einschränkt.

Ballonkatheter werden aus sehr dünnen Kunststoffschläuchen durch Aufweitung eine Segments von 1 bis ca. 10 cm Länge geformt. Die aufgeweitete, sehr dünnwandige Ballonmembran wird anschließend in mehrere längs zur Katheterachse angeordnete Falten gelegt und fest um die Katheterachse gewickelt, so dass der aufgeweitete Bereich im gefalteten Zustand einen nur minimal größeren Durchmesser aufweist als der übrige Katheter. Die enge Faltung der Ballonhülle ist Voraussetzung für die problemlose Passage des Ballonkatheters durch Einführungsschleusen, Führungskatheter und z.B. stark verengte Abschnitte von Blutgefäßen.

Die Ballone von Kathetern können in gefaltetem und entfaltetem Zustand beschichtet werden, wobei in jedem Fall eine intakte, ausreichend gleichmäßige Beschichtung der Oberfläche erzielt wird und die Wirkstoffe auch während des Einfaltens eines im entfalteten Zustand beschichteten Ballonkatheters ausreichend fest an dessen Oberfläche haften.

Die Herstellung eines in entfaltetem Zustand beschichteten Ballons erfolgt ohne Beeinträchtigung der Beschichtung beispielsweise durch die Verwendung von Ballonhüllen mit vorgeformten Falten und Biegungen, deren Struktur im Material durch das Aufdehnen nicht verloren geht und die nach Ablassen des Druckes aus dem Ballon bewirken, dass sich die Ballonhülle wieder regelrecht wenigstens lose einfaltet, ohne dass es einer äußeren Kraft als primäre Ursache bedarf. Erst danach werden die vorgeformten Falten von außen oder durch Vakuum zusammengepresst. In keinem Falle sind Falten zum Festhalten des Wirkstoffs erforderlich. Weiterhin kann die Einfaltung durch geringe mechanische Kräfte mittels sehr glatter Materialien bewirkt werden, wobei die Werkzeuge auch beispielsweise mit schlüpfrigen biokompatiblen Flüssigkeiten benetzt sein können, in denen sich die Wirkstoffe nicht oder jedenfalls nicht gut lösen.

Gemäß einer weiteren Erfindungsvariante werden die Ballone fertig gefalteter Ballonkatheter durch Tauchen in niedrig viskose Wirkstofflösungen beschichtet. Dabei dringen Lösungsmittel und Wirkstoff zwischen die extrem engen Falten und bilden dort einen überraschend gleichmäßigen und in der Dosis reproduzierbaren Belag, der durch keinen weiteren Arbeitsschritt beschädigt wird. Die außen anhaftende Lösung bzw. der nach Trocknen des Lösungsmittels außen anhaftende Belag kann dort belassen oder in einem weiteren Arbeitsschritt entfernt werden, so dass nur der von den Falten des Ballons verdeckte Wirkstoff erhalten bleibt.

Nach der Beschichtung kann bei gefaltetem Ballon ein Stent auf den Ballonkatheter geschoben und auf diesem festgepresst werden. Danach ist nur noch die Sterilisation, z. B. mittels Ethylenoxid, erforderlich.

Der so gestaltete Arbeitsgang ist außerordentlich einfach, wenig störanfällig und auch mit mechanisch, chemisch und physikalisch empfindlichen Beschichtungsmaterialien durchzuführen. Es hat sich gezeigt, dass die Beschichtung nach diesem Verfahren zu keiner unerwünschten Lockerung oder Verklebung der Faltung führt und dass der derart aufgetragene Wirkstoff fest genug haftet, um auf dem Weg durch das Blut nicht abgetragen zu werden, andererseits bei Inflation des Ballons im Zielgewebe den Wirkstoff weitgehend freigibt.

Als Arzneistoffe kommen stark lipophile, weitgehend wasserunlösliche, an beliebige Gewebebestandteile bindende stark wirksame Arzneistoffe in Frage. Als lipophil werden Arzneistoffe bezeichnet, deren Verteilungskoeffizient Butanol: wässriger Puffer pH 7 = 0.5, bevorzugt = 1 und besonders bevorzugt = 5 bzw. Octanol: wässriger Puffer pH 7 = 1, bevorzugt = 10, besonders bevorzugt > 50 ist. Alternativ oder zusätzlich sollen die Arzneistoffe zu > 10 %, bevorzugt zu > 50 %, besonders bevorzugt zu > 80 % reversibel und/oder irreversibel an Zellbestandteile binden. Bevorzugt sind Substanzen zur Hemmung der Zellproliferation oder auch entzündlicher Prozesse oder Antioxidantien wie Paclitaxel und andere Taxane, Rapamycin und verwandte Substanzen, Tacrolimus und verwandte Substanzen, Corticoide, Sexualhormone (Östrogen, Estradiol, Antiandrogene) und verwandte Substanzen, Statine, Epothilone, Probucol, Prostacycline, Angiogeneseinduktoren etc.

Die Substanzen liegen bevorzugt als trockene Festsubstanz auf den Oberflächen der unterschiedlichen Medizinprodukte vor. Bevorzugt sind Partikel geringster Größe (in der Mehrzahl < 5 *µ*m, bevorzugt < 1 *µ*m, besonders bevorzugt < 0.1 *µ*m), besonders bevorzugt sind amorphe, nicht kristalline Strukturen feinster Partikelgröße, die bei Kontakt mit Gewebe wegen ihrer großen Oberfläche trotz grundsätzlich geringer Wasserlöslichkeit der Arzneistoffe rasch in Lösung gehen und nicht als Mikrokapseln fungieren, d. h. sich spontan und schnell lösen. Dabei ist es ausreichend, dass eine wirksame Dosis in Form kleinster oder amorpher Partikel vorliegt; größere Partikel tragen zwar zur Wirkstoffkonzentration im Gewebe kaum bei, stören aber auch nicht. Erfindungsgemäß liegt der Wirkstoff in Form von amorphen Partikeln vor. Die Dosierung richtet sich nach der erwünschten Wirkung und der Wirksamkeit des ver-wendeten Arzneistoffes. Sie kann bis zu 5 *µ*g/ mm² erreichen, wobei dies keine Obergrenze darstellt. Geringere Dosierungen sind leichter zu realisieren.

Die Beschichtung der Medizinprodukte erfolgt mittels Lösungen, Suspensionen oder Emulsionen der genannten Arzneistoffe. Geeignete Lösungs- Suspendier- oder Emulsionsmedien sind beispielsweise Ethanol, Isopropanol, Ethylacetat, Diethylether, Aceton, Dimethylsulfoxid, Dimethylformamid, Glycerin, Wasser oder Mischungen derselben. Die Auswahl der Lösungsmittel folgt entsprechend der Löslichkeit der Wirkstoffe und Zusatzstoffe sowie der Benetzung der zu beschichtenden Oberflächen und der Wirkung auf die Struktur der nach Verdampfen der Lösungsmittel zurückbleibenden Beschichtung und Partikel, deren Haftung auf der Oberfläche und die Wirkstoffübertragung in das Gewebe während sehr kurzer Kontaktzeiten.

Die Auftragung kann beispielsweise durch Tauchen, Bestreichen, Auftragen mittels Volumenmesseinrichtungen oder Besprühen jeweils bei unterschiedlichen Temperaturen und ggf. Dampfsättigungen der Lösungsmittel in der Atmosphäre geschehen. Der Vorgang kann mehrfach, ggf. auch unter Verwendung verschiedener Lösungsmittel und Hilfsstoffe, wiederholt werden.

Die Ballone fertig gefalteter Ballonkatheter lassen sich durch Tauchen in wirkstoffhaltige Lösungen oder andere Maßnahmen erstaunlich gleichmäßig, reproduzierbar, in der Dosis steuerbar und ohne Beeinträchtigung der Funktion der Katheter beschichten. Bei wiederholtem Tauchen in ungesättigten Wirkstofflösungen kommt es wider Erwarten nicht zu einer vollständigen Ablösung des vorher aufgetragenen Wirkstoffs sondern zu einer reproduzierbaren Erhöhung des Wirkstoffgehaltes der Ballone.

Überschüssige Lösung bzw. überschüssige, außen lose anhaftende Substanzen aus der Beschichtungslösung können mit einfachen Methoden entfernt werden, ohne dass es zu einer Beeinträchtigung der Wirksamkeit der Beschichtung kommt.

Die erfindungsgemäß ausgebildeten und hergestellten Ballonkatheter kommen kurzzeitig, d. h. für Sekunden, Minuten oder wenige Stunden mit dem Gewebe in Kontakt. In einigen Fällen ist es erwünscht, das Gewebe in unmittelbarer Nähe des Medizinproduktes pharmakologisch zu behandeln, zum Beispiel an überschießendem Wachstum als Reaktion auf eine Verletzung zu hindern oder Tumorwachstum zu reduzieren oder die Einsprossung von Blutgefäßen zu fördern oder Entzündungsreaktionen zu vermindern. In all diesen Fällen kann mit dem oben beschriebenen Verfahren eine hohe lokale Arzneistoffkonzentration für erstaunlich lange Zeit erzielt werden. Ein wesentlicher Vorteil ist die außerordentliche Vielfalt der Einsatzmöglichkeiten der beschriebenen Produkte und Verfahren.

Eine bevorzugte Anwendung ist die Verminderung der durch die Gefäßdilatation mittels Ballonkathetern induzierte Hyperproliferation der Gefäßwände. Diese ist im Bereich gegebenenfalls implantierter Gefäßstützen (Stents) auch durch Beschichtung der Stents mit Arzneistoffen zu erzielen, allerdings nur im unmittelbar durch den Stent bedeckten Gefäßbereich. Die beschichteten Ballonkatheter behandeln darüber hinaus die behandlungsbedürftigen Bereiche kurz vor und kurz hinter dem Stent, sie können ohne erneute Stentimplantation den Bereich innerhalb bereits vorhandener Stents behandeln oder Gefäße, in die kein Stent implantiert werden soll oder kann. Vorteilhaft gegenüber den über einen langen Zeitraum Arzneistoff freisetzenden Stents ist die bessere Einheilung bei gleichzeitig guter Hemmung der Hyperproliferation und das geringere Thromboserisiko.

Nachfolgend werden mehrere Ausführungsformen am Beispiel der Beschichtung von Ballonkathetern sowie in Bezug auf die Haftung der Beschichtung im Blut, die Restenosehemmung und den Wirkstoffgehalt der Katheter beschrieben. Diese Beispiele sind Referenzbeispiele und sind nicht auf den beanspruchten Gegenstand beschränkt.

### Beispiel 1:

### Beschichten eines expandierten Ballonkatheters mit Paclitaxel in Ethylacetat

Ballonkatheter der Fa. BMT, Oberpfaffenhofen/ München, Deutschland, mit der Bezeichnung Joker Lite, Ballongröße 2.5 mm x 20 mm, werden nach maximaler Expansion für 1 min. über die gesamte Ballonlänge in Ethylacetat, 18.8 mg Paclitaxel/ ml, + 1% pharmazeutisches Olivenöl, getaucht, getrocknet:
Paclitaxelgehalt 39 *µ*g (nach Extraktion mit Ethanol, HPLC).

### Beispiel 2:

### Beschichten eines gefalteten Ballonkatheters mit Paclitaxel in Ethylacetat

Ballonkatheter der Fa. BMT, Oberpfaffenhofen/ München, Deutschland, mit der Bezeichnung Joker Lite, Ballongröße 2.5 mm x 20 mm, werden im gefalteten Zustand für 1 min. über die gesamte Ballonlänge in Ethylacetat, 18.8 mg Paclitaxel/ ml, + 1% pharmazeutisches Olivenöl, getaucht, getrocknet:
Paclitaxelgehalt 69 *µ*g.

### Beispiel 3:

### Beschichten eines gefalteten Ballonkatheters mit Paclitaxel in Ethylacetat

a) Ballonkatheter der Fa. BMT, Oberpfaffenhofen/ München, Deutschland mit der Bezeichnung Joker Lite, Ballongröße 2.5 mm x 20 mm, werden im gefalteten Zustand für 1 min. über die gesamte Ballonlänge in Ethylacetat, 16.6 mg Paclitaxel/ ml, getaucht, 4 h getrocknet: Paclitaxelgehalt 54 *µ*g
b) Ebenso, jedoch noch 2 mal 5 sec. mit 1 h Trocknungszeit nach jedem Tauchvorgang in Lösung A (= 3.33 ml Ethylacetat + 100.0 mg Paclitaxel) getaucht: Paclitaxelgehalt 126 *µ*g
c) Ebenso, jedoch noch 4 mal 5 sec. mit 1 h Trocknungszeit nach jedem Tauchvorgang in die gleiche Lösung getaucht: Paclitaxelgehalt 158 *µ*g

### Beispiel 4:

### Beschichten eines Ballonkatheters mit Paclitaxel in Aceton

350 mg Paclitaxel in 9.0 ml Aceton lösen; Ballonkatheter der Fa. BMT, Oberpfaffenhofen/ München, Deutschland mit der Bezeichnung Joker Lite, Ballongröße 2.5 mm x 20 mm, werden im maximal expandierten Zustand für 1 min. über die gesamte Ballonlänge getaucht, entnommen, das Lösungsmittel wird bei Raumtemperatur 12 h getrocknet. Danach wird der Ballon deflatiert und mit PTFE-beschichtetem Werkzeug in üblicher Weise eingefaltet. Optional kann ein Stent geeigneter Größe auf den Ballon gecrimmt werden: 29 *µ*g Paclitaxel auf dem Ballon.

### Beispiel 5:

### Beschichten eines Ballonkatheters mit Paclitaxel in Aceton

a) Tauchen gefaltete Ballonkatheter der Fa. BMT mit der Bezeichnung Allegro, Ballongröße 2.5 x 20 mm in ein Gemisch von 0.15 ml Ethanol + 4.5 *µ*l Ultravist 300 ( Röntgenkontrastmittel der Schering AG, Berlin, Deutschland) + 1.35 ml Aceton + 0.8 mg Sudanrot + 30.0 mg Paclitaxel:
   Die gefalteten Ballonabschnitte der Katheter werden 5 x getaucht, das 1. Mal für 1 Minute, dann 3 h Trocknungszeit, danach 4 mal für je 5 sec im Abstand von 1 h; danach wurde ein Stent aufgecrimpt und der Katheter mit Stent in üblicher Weise mit Ethylenoxid sterilisiert: Paclitaxelgehalt 172 *µ*g, keine mittels HPLC detektierbaren Zersetzungsprodukte des Wirkstoffs
b) Statt Ultravist 300 wird eine gesättigte wässrige Mannit-Lösung zugesetzt
c) Statt Ultravist 300 wird eine gesättigte wässrige Natrium-Salicylat Lösung, pH 7.5, zugesetzt.
d) Der fertigen Lösung nach (5a) werden 5 mg Acetylsalicylsäure zugesetzt.
e) Der fertigen Lösung nach (5a) werden 5 mg Glycerin zugesetzt

### Beispiel 6:

### Haftung des Wirkstoffs in Blut

Es wurden 12 Ballonkatheter der Fa. BMT mit der Bezeichnung Allegro, Ballongröße 2.5 x 20 mm, verwendet. Je 6 Stück der gefalteten Ballonabschnitte der Katheter wurden entweder in [0.15 ml Ethanol + 4.5 *µ*l Ultravist 300 + 1.35 ml Aceton + 0.8 mg Sudanrot+ 30.0 mg Paclitaxel] oder in [1.5 ml Ethylacetat + 0.8 mg Sudanrot + 31.0 mg Paclitaxel] 5 x getaucht, das 1. Mal für 1 Minute, dann 3 h Trocknungszeit, danach 4 mal für je 5 sec im Abstand von 1 h; danach wurden je 3 gefaltete Ballone jeder Serie in 50 ml Humanblut 5 min bei 37 °C leicht bewegt und dann zur Analyse des Paclitaxel-Gehaltes entnommen: Verminderung der Mittelwerte (n=3 je Beschichtungsmethode) durch 5 Minuten Bewegung in Blut im Vergleich zu je 3 Kontrollkathetern, die nicht in Blut inkubiert wurden.

| | |
|---|---|
| Aceton: | 12 % |
| Ethylacetat: | 10 % |

### Beispiel 7:

### Untersuchung der Restenosehemmung nach Angioplastie und Stentimplantation an den Koronarien von Schweinen

Gefaltete Ballonkatheter der Fa. BMT Typ Joker Lite, BMT 3.5 x 20 mm oder 3.0 x 20 mm wurden entweder in
Lösung A) 3.33 ml Ethylacetat (EA)+ 100.0 mg Paclitaxel oder in
Lösung B) 0.45 ml Ethanol + 100 *µ*l Ultravist-370 + 4.5 ml Aceton (Ac) + 150.0 mg Paclitaxel
für 1 min eingetaucht und bei Raumtemperatur über Nacht getrocknet. Ein weiterer (niedrige Dosis = L) bzw. 4 weitere (hohe Dosis = H) Tauchvorgänge wurden jeweils nur für 5 Sekunden am nächsten Tag im Abstand von 1 h durchgeführt.

Wirkstoffgehalt nach 2 maligem Tauchen in Lösung (B) im Mittel 250 *µ*g, bei 5 maligem Tauchen in Lösung (B) 500*µ*g, in Lösung (A) 400 *µ*g.

Insgesamt 22 Schweinen wurde mittels der mit Paclitaxel beschichteten Katheter oder mittels unbeschichteter Katheter Stents in die linke Vorderwand- oder Seitenwand-Koronararterie implantiert, wobei die Gefäße zur Stimulation der Restenose durch Gewebehyperplasie leicht überdehnt wurden. Nach 5 Wochen wurden die Tiere reangiographiert und das Maß der Gefäßverengung auf den Angiogrammen mit einem automatischen Computerprogramm gemessen.

| Gruppe | Stenose (%) |
|---|---|
| Unbeschichtet | 50.49 |
| AcL | 20.22 |
| EAH | 36.01 |
| AcH | 0.86 |
| p | .004 |

Quantitative Koronarangiographie 5 Wochen nach Stentimplantation mit unbeschichteten und beschichteten Kathetern; Stenose = prozentuale Verminderung des Lumendurchmessers im Bereich des Stents im Vergleich zu dem Lumendurchmesser unmittelbar nach Stentimplantation Mittelwert und statistische Signifikanz des Behandlungseffekts.

### Beispiel 8:

### Wirkstoffgehalt der Katheter nach Gefäßdilatation und Stentimplantation

Die Ballone aus Beispiel 8 wurden nach Stentimplantation und Entnahme aus den Tieren auf ca. 3 cm Länge von den Ballonkathetern abgetrennt und in 1.5 ml Ethanol überführt. Der Paclitaxelgehalt wurde mittels HPLC bestimmt. Alle verfügbaren beschichteten Ballone und eine Auswahl unbeschichteter Ballone wurden untersucht.
Koronar,

| | | | |
|---|---|---|---|
| 3.0 x 20 mm, Beschichtung: | Ac hoch | 38 ± 4 *µ*g | (n=4) |
| | Ac niedrig | 22 ± 5 *µ*g | (n=2) |
| | EEE hoch | 41 | (n=1) |
| 3.5 x 20 mm, Beschichtung: | Ac hoch | 37 ±10 *µ*g | (n=8) |
| | Ac niedrig | 26 ± 6 *µ*g | (n=8) |
| | EEE hoch | 53 ± 9 *µ*g | (n=9) |
| Unbeschichtet (unabhängig von Größe und Gefäßgebiet) | | 0.9 ± 1.0 *µ*g | (n=7) |

Aus Beispiel 6 ergibt sich, dass maximal 10 % der Dosis verloren gehen bevor der Ballon expandiert wird und ca. 10 % der Dosis auf dem Ballon verbleiben.

### Beispiel 9 :

Probucol wird in einer Konzentration von 100 mg/ ml in Aceton eingebracht; die Lösung wird wie in den vorhergehenden Beispielen beschrieben zum Beschichten der Ballonkatheter eingesetzt.

### Beispiel 10:

Rapamycin wird in einer Konzentration von 10 mg/ ml in Diethylether gelöst. Die Beschichtung der Ballonanteile der Katheter erfolgt wie in den vorhergehenden Beispielen beschrieben; die Ballone sollen nach der Entnahme aus der Beschichtungslösung möglichst sofort horizontal ausgerichtet und ständig um ihre Längsachse gedreht werden.

### Beispiel 11:

Epothilon B wird in einer Konzentration von 2 mg/ ml in Ethylacetat gelöst; die Lösung wird wie in den vorhergehenden Beispielen beschrieben zum Beschichten der Ballonkatheter eingesetzt.

## Patentansprüche

1. Ballonkatheter zur Arzneimittelabgabe für die selektive Therapie bestimmter erkrankter Gewebeabschnitte oder Organteile umfassend einen Ballon, **dadurch gekennzeichnet, dass** der Ballon aus einem Material besteht, welches ausgewählt ist aus Polyamiden, Polyamid-Gemischen und Copolymeren, Polyethylenterephthalat, Polyethylen und Copolymeren, Polyurethan und Naturkautschuk, und dass an der Oberfläche des zumindest kurzzeitig das erkrankte Gewebe unter Druck kontaktierenden Ballons ein stark lipophiler, weitgehend wasserunlöslicher, an beliebige Gewebebestandteile bindender Wirkstoff mit nach Gewebekontakt sofortiger Wirkstofffreigabe haftet, wobei der Wirkstoff in Form von amorphen Partikeln vorliegt, erhältlich durch das Auftragen einer Lösung bestehend aus dem Wirkstoff und einem Lösungsmittel auf die Oberfläche des Ballons und Verdampfen des Lösungsmittels.

2. Ballonkatheter nach Anspruch 1, **dadurch gekennzeichnet, dass** der Wirkstoff ausgewählt ist aus der Gruppe umfassend Paclitaxel und anderen Taxanen, Rapamycin, Tacrolimus, Corticoiden, Sexulahormonen, Statinen, Epothilonen, Probucol, Prostacyclinen und Angiogeneseinduktoren.

3. Ballonkatheter nach Anspruch 2, **dadurch gekennzeichnet, dass** an der Oberfläche des Ballons der Wirkstoff in einer Dosierung bis zu 5 µg/mm² vorliegt.

4. Ballonkatheter nach einem der vorherigen Ansprüche, **dadurch gekennzeichnet, dass** die zu beschichtende Oberfläche glatt, aufgeraut oder strukturiert ist.

5. Ballonkatheter nach einem der Ansprüche 2-4, **dadurch gekennzeichnet, dass** der Wirkstoff in Form von amorphen Partikeln mit einer Größe von 0,1 µm bis <5 µm vorliegt.

6. Ballonkatheter nach einem der vorherigen Ansprüche, **dadurch gekennzeichnet, dass** der Ballon einen Durchmesser von 2-4 mm und eine Länge von 1,0 - 4,0 cm aufweist.

7. Ballonkatheter nach einem der vorherigen Ansprüche in Verbindung mit einem Stent.

8. Ballonkatheter nach einem der vorherigen Ansprüche, wobei das Lösungsmittel Ethanol, Isopropanol, Ethylacetat, Diethylether, Aceton, Dimethylsulfoxid, Dimethylformamid, Glycerin und/oder Wasser umfasst.

9. Ballonkatheter nach einem der vorherigen Ansprüche, wobei das Auftragen durch Tauchen, Bestreichen, Auftragen mittels einer Volumenmesseinrichtung oder Besprühen erfolgt.

10. Ballonkatheter nach einem der vorherigen Ansprüche, wobei das Auftragen auf den Ballon im gefalteten Zustand erfolgt, oder wobei das Auftragen auf den Ballon im expandierten Zustand erfolgt.

## Claims

1. Balloon catheter for delivery of medication for the selective therapy of particular diseased tissue sections or organ parts comprising a balloon, **characterized in that** the balloon consists of a material selected from polyamides, polyamide-blends and copolymers, polyethylenterephthalate, polyethylene and copolymers, polyurethane, and natural rubber, and that a strongly lipophilic, largely water insoluble drug that binds to any tissue parts adheres to the surface of the balloon that contacts diseased tissue at least shortly under pressure with immediate release of the drug upon tissue contact, wherein the drug is present in the form of amorphous particles, obtainable by applying a solution consisting of the drug and a solvent to the surface of the balloon and evaporating the solvent.

2. Balloon catheter according to claim 1, **characterized in that** the drug is selected from the group comprising paclitaxel and other taxanes, rapamycin, tacrolimus, corticoids, sexual hormones, statins, epothilones, probucol, prostacycline and angiogenesis inducers.

3. Balloon catheter according to claim 2, **characterized in that** the drug is present on the surface of the balloon in a doses of up to 5 µg/mm².

4. Balloon catheter according to any of the preceding claims, **characterized in that** the surface to be coated is smooth, roughened or structured.

5. Balloon catheter according to any of claims 2-4, **characterized in that** the drug is present in the form of amorphous particles having a size of 0.1 µm to <5 µm.

6. Balloon catheter according to any of the preceding claims, **characterized in that** the balloon has a diameter of 2-4 mm and a length of 1.0-4.0 cm.

7. Balloon catheter according to any of the preceding claims in conjunction with a stent.

8. Balloon catheter according to any of the preceding claims, wherein the solvent comprises ethanol, isopropanol, ethyl acetate, diethyl ether, acetone, dimethyl sulfoxide, dimethyl formamide, glycerine and/or water.

9. Balloon catheter according to any of the preceding claims, wherein applying is conducted by dipping, painting, applying with a volume measuring device or by spraying.

10. Balloon catheter according to any of the preceding claims, wherein the application onto the balloon is conducted in the deflated state, or wherein the application onto the balloon is conducted in the expanded state.

## Revendications

1. Cathéter à ballonnet pour l'administration de médicament pour la thérapie sélective de portions de tissus malades ou parties d'organe déterminées, **caractérisé en ce que** le ballonnet est constitué d'un matériau, qui est sélectionné parmi les polyamides, mélanges de polyamides et copolymères, polyéthylène téréphtalate, polyéthylène et copolymères, polyuréthane et caoutchouc naturel et **en ce que** sur la surface du ballonnet contactant au moins pendant une courte durée le tissu malade sous pression, un principe actif fortement lipophile, dans une large mesure insoluble dans l'eau, se liant à des composants du tissu au choix adhère avec une administration de principe actif immédiate après le contact avec le tissu, dans lequel le principe actif se présente sous forme de particules amorphes, obtenu par application d'une solution constituée d'un principe actif et un solvant sur la surface du ballonnet et par évaporation du solvant.

2. Cathéter à ballonnet selon la revendication 1, **caractérisé en ce que** le principe actif est sélectionné dans le groupe comprenant le paclitaxel et d'autres taxanes, le rapamycine, le tacrolimus, les corticoïdes, les hormones sexuelles, les statines, les épothilones, le probucol, la prostacycline et les inducteurs d'angiogenèse.

3. Cathéter à ballonnet selon la revendication 2, **caractérisé en ce que** sur la surface du ballonnet le principe actif est présent dans un dosage allant jusqu'à 5 µg/mm².

4. Cathéter à ballonnet selon une des revendications précédentes, **caractérisé en ce que** la surface à revêtit est lisse, rugueuse ou structurée.

5. Cathéter à ballonnet selon une des revendications 2-4, **caractérisé en ce que** le principe actif est présent sous forme de particules amorphes avec une taille de particule comprise entre 0,1 µm jusqu'à < 5 µm.

6. Cathéter à ballonnet selon une des revendications précédentes, **caractérisé en ce que** le ballonnet présente un diamètre de 2-4 mm et une longueur de 1,0-4,0 cm.

7. Cathéter à ballonnet selon une des revendications précédentes en liaison avec un stent.

8. Cathéter à ballonnet selon une des revendications précédentes, dans lequel le solvant comprend de l'éthanol, isopropanol, éthylacétate, diéthyléther, acétone, diméthylsulfoxide, diméthylformamide, glycérine et/ou eau.

9. Cathéter à ballonnet selon une des revendications précédentes, dans lequel l'application a lieu par immersion, enduction, application au moyen d'un dispositif de mesure de volume ou pulvérisation.

10. Cathéter à ballonnet selon une des revendications précédentes, dans lequel l'application sur le ballonnet a lieu en l'état plié ou dans lequel l'application sur le ballonnet a lieu en l'état expansé.
